# EUROPÄISCHE PATENTSCHRIFT

(11) **EP 4 176 914 B1**
(45) Veröffentlichungstag und Bekanntmachung des Hinweises auf die Patenterteilung: **16.07.2025**
(21) Anmeldenummer: 22198780.3
(22) Anmeldetag: 29.09.2022
(51) Int. Cl.: A61M 16/04

(54) **BEATMUNGSVORRICHTUNG MIT LEITUNG ZUM BEFÜLLEN EINES CUFFS IM TRACHEALABSCHNITT**
BREATHING APPARATUS WITH CONDUIT FOR FILLING A CUFF IN THE TRACHEAL SECTION
DISPOSITIF RESPIRATOIRE AVEC CONDUIT POUR REMPLIR UN BALLONNET DANS LA PARTIE TRACHÉE

(30) Priorität: 03.11.2021 DE 102021128640
(43) Veröffentlichungstag der Anmeldung: 10.05.2023
(73) Patentinhaber: Coloplast A/S, 3050 Humlebaek (DK)
(72) Erfinder: Schnell, Ralf, 63500 Seligenstadt (DE)

(56) Entgegenhaltungen:
- WO-A1-2021/059183
- WO-A1-2022/144355
- WO-A1-2022/144357
- DE-A1- 102017 131 172

## Beschreibung

Die vorliegende Erfindung betrifft eine Beatmungsvorrichtung, wie z.B. eine Tracheostomiekanüle, mit einem Kanülenrohr und einer aufpumpbaren Manschette. Diese Manschette erstreckt sich im Trachealabschnitt an der Außenseite der Rohrwand ringförmig um das Kanülenrohr und weist ein variables Lumen zur Aufnahme eines über eine Leitung und durch eine Öffnung in der Manschette zugeführten Fluids auf.

Viele Tracheostomiekanülen bzw. Endotrachealkanülen sind im Trachealabschnitt mit einer Manschette, die auch Cuff genannt wird, ausgestattet. Die Manschette bzw. der Cuff wird typischerweise von einer um das Kanülenrohr angeorderneten Kunststofffolie gebildet, die im nicht aufgepumpten Zustand beispielsweise eine Wandstärke im Bereich von 5 bis 300 µm aufweisen kann.

Die WO2021/059183 A1 offenbart eine Beatmungsvorrichtung mit einer aufpumpbaren Manschette im Trachealabschnitt und einer variablen Fluid-Leitung.

Die Manschette bzw. der Cuff ist mit Luft oder einem anderen Fluid aufpumpbar und befindet sich im Trachealabschnitt in distaler Richtung kurz vor dem Patientenende des Kanülenrohrs. Das Lumen der Manschette ist dahingehend variabel, dass es sich vergrößert, wenn in das Lumen der Manschette ein Fluid gepumpt wird, und dass es sich verkleinert, wenn im Lumen der Manschette befindliches Fluid wieder herausgelassen wird. Der Querschnitt der Manschette nimmt mit dem Querschnitt von deren Lumen zu und ab, wobei die Differenz zwischen dem Querschnitt der Manschette und dem Querschnitt von deren Lumen angesichts der geringen Wandstärke der Kunststofffolie, aus der die Manschette besteht, vernachlässigbar ist.

Die Manschette bzw. der Cuff hat die Aufgabe, die Luftröhre um das Kanülenrohr herum abzudichten. Dies wird dadurch erreicht, dass die Manschette bis zu einem Fülldruck (Überdruck bezogen auf den Umgebungs- bzw. Atmosphärendruck) aufgepumpt wird, bei dem die Ausdehnung der Manschette so groß wird, dass die Manschette den Bereich zwischen Luftröhre und Kanülenrohr ausfüllt. Sobald der Querschnitt der Manschette groß genug ist, um den Bereich zwischen Luftröhre und Kanülenrohr auszufüllen und abzudichten, spricht man von einem "geblockten" Cuff.

Auf diese Weise wird einerseits sichergestellt, dass bei künstlich beatmeten Patienten die von einem Beatmungsgerät zur Verfügung gestellte Beatmungsluft vollständig in die Bronchien gelangt und von dort auch wieder definiert den Patienten verlässt. Würde der Cuff nicht ausreichend abdichten, könnten signifikante Mengen Beatmungsluft unkontrolliert durch die natürlichen Atemwege oder das Tracheostoma entweichen. In diesem Fall würde das Beatmungsgerät Alarm geben.

Andererseits hat ein Cuff auch die Aufgabe zu verhindern, das Sekret aus dem subglottischen Bereich in die Bronchien gelangt. Dies könnte nämlich zu sogenannten "Ventilations-assoziierten Pneumonien (VAP)" führen.

Der spezifische Fülldruck, der zum Blocken eines Cuffs erforderlich ist, hängt u.a. von der Größe und Bauart der Kanüle ab. Bei den sogenannten "High Volume Low Pressure"-Cuffs, die mit Luft befüllt werden und sich unter Faltenbildung an die Trachealwand anlegen, liegt der spezifisch Fülldruck zum Blocken des Cuffs der Kanüle typischerweise im Bereich von 15 bis 30 mbar. Bei den sogenannten "Low Volume High Pressure"-Cuffs, die sich beim Befüllen elastisch aufweiten wie ein Luftballon, liegt der spezifische Fülldruck typischerweise im Bereich von 60 bis 200 mbar.

Damit ein Cuff seine abdichtende Eigenschaft entfalten kann, muss er mit einer ausreichenden Fluidmenge (typischerweise Luft) befüllt werden. Die Befüllung erfolgt entweder über einen Füllschlauch, der mit einem Ende in das Lumen des Cuffs mündet, während das andere Ende proximal bis in den extrakorporalen Bereich der Beatmungsvorrichtung reicht. Alternativ kann die Zuführung des Fluids zum Cuff wenigstens abschnittsweise auch über eine in der Rohrwand des Kanülenrohrs verlaufende Cuff-Leitung erfolgen. Der Begriff Cuff-Leitung schließt beide Ausführungsformen ein.

Aus baulichen Gründen ist die Cuff-Leitung im extrakorporalen Bereich häufig anders ausgeführt als im intrakorporalen Bereich. Beispielsweise kann die Cuff-Leitung im intrakorporalen Bereich in Form einer in der Rohrwand des Kanülenrohrs verlaufenden Leitung vorgesehen sein und im extrakorporalen Bereich in Form eines außerhalb des Kanülenrohres geführten separaten Füllschlauchs vorgesehen sein.

Die Zuführung des Fluids erfolgt über ein Füllventil, das am extrakorporalen Ende der Cuff-Leitung bzw. am Kontrollballon angebracht ist. Zur Befüllung des Cuffs kann an dieses Ventil beispielsweise eine Spritze oder ein eigens dafür vorgesehenes Gerät angeschlossen werden.

Der Innendurchmesser eines handelsüblichen Füllschlauchs bzw. einer in der Rohrwand vorgesehenen Cuff-Leitung beträgt im intrakorporalen Bereich typischerweise zwischen 0,5-1 mm. Prinzipiell wäre es aber aus verschiedenen Gründen wünschenswert Cuff-Leitungen mit deutlich größeren Innendurchmessern zu haben. Dies würde beispielsweise das Risiko reduzieren, dass sich eventuell im Füllschlauch vorhandenes Kondenswasser bei der Cuffdruckeinstellung aufgrund der hier wirkenden Kapillarkräfte bei den üblicherweise sehr niedrigen Druckdifferenzen von meist nicht mehr als 30 mbar nicht in Bewegung setzen lässt. In diesen Fällen kann es dazu kommen, dass Wasser die Zuleitung blockiert, wodurch der für die Druckmessung erforderliche Druckausgleich zwischen dem proximal zur Druckermittlung angeordneten Manometer und dem Cuff-Lumen verhindert wird. Liegt eine solche Situation vor führt die Cuffdruckkontrolle zu falschen Ergebnissen, da man nur den Druck messen kann, der vor der Blockade herrscht und nicht den Druck, der hinter der Blockade im Cuff vorliegt.

Ein weiterer Nachteil eines engen Durchmessers der Cuff-Zuleitung besteht darin, dass der Druckausgleich zwischen dem Druck am Füllventil und dem Druck im Cuff-Lumen oft relativ träge abläuft. Dies ist insbesondere bei der Verwendung von aktiv gesteuerten Cuffdruckvorrichtungen nachteilig.

Der Innendurchmesser der Cuff-Leitung ist jedoch insbesondere im intrakorporalen Bereich auch nicht beliebig groß zu wählen. Hierzu sind die sterischen Verhältnisse in der Luftröhre zu eng, was der Hauptgrund dafür ist, dass handelsübliche Füllschläuche einen möglichst geringen Durchmesser haben oder die Cuff-Leitung sogar in die Rohrwand des Kanülenrohres integriert wird, um auf diese Weise den Querschnitt der Beatmungsvorrichtung im Trachealabschnitt insgesamt möglichst gering zu halten. Da man aus sterischen Gründen die Wandstärke des Kanülenrohres auch nicht beliebig groß gestalten kann, sind hier ebenfalls enge Limits bezüglich der in die Kanülenwand integrierten Cuff-Leitung gesetzt. Die Herstellung eines Kanülenrohres mit einer in der Rohrwand integrierten Cuff-Leitung ist zudem bei Kanülenrohren, die mit dem Spritzgussverfahren hergestellt werden, ausgesprochen schwierig und teuer, da hierfür spezielle Spritzgusswerkzeuge angefertigt werden müssen.

Die Motivation für die vorliegende Erfindung bestand also darin, eine Beatmungsvorrichtung, wie z.B. eine Tracheostomiekanüle oder eine Endotrachealkanüle, bereitzustellen, welche die Nachteile, die mit entsprechende Vorrichtungen verbunden sind, die eine Cuff-Leitung mit relativ geringem Innenvolumen aufweisen, überwindet und dabei die schwierigen sterischen Verhältnisse in der Trachea berücksichtigt.

Die Erfindung wird in den angehängten Ansprüchen definiert.

Erfindungsgemäß wird dies durch eine Beatmungsvorrichtung erreicht, die ein Kanülenrohr aufweist, dessen Rohrwand einen Extrakorporalabschnitt, einen Stomaabschnitt und einen Trachealabschnitt aufweist und eine aufpumpbare Manschette, die sich im Trachealabschnitt an der Außenseite der Rohrwand ringförmig um das Kanülenrohr erstreckt, wobei die Manschette ein variables Lumen zur Aufnahme eines Fluids aufweist, wobei die Beatmungsvorrichtung außerdem eine Fluid-Leitung mit einer Öffnung in das Lumen der Manschette aufweist, wobei die Beatmungsvorrichtung insbesondere dadurch gekennzeichnet ist, dass die Fluid-Leitung im Bereich des Stomaabschnitts und/oder des Trachealabschnitts zumindest abschnittsweise von einer auf der Außenseite des Kanülenrohres angeordneten Folie gebildet wird, die ein variables Lumen für die Durchführung von Fluid ausbildet, wobei das variable Lumen eines von der Folie gebildeten Abschnitts n der Fluid-Leitung bei einem spezifischen Fülldruck zum Blocken der Manschette im Bereich von 15-200 mbar Überdruck bezogen auf den Umgebungsdruck (Atmosphärendruck) einen Querschnitt Qn₂ aufweist, der wenigstens um den Faktor 3 größer ist als der Querschnitt Qn₁, der vorliegt, wenn in der Manschette bezogen auf den Umgebungsdruck (Atmosphärendruck) ein Unterdruck von -30 mbar vorliegt.

Ein Unterdruck von -30 mbar wird leicht erreicht, wenn man den Cuff, wie üblich, mit Hilfe einer Spritze entleert. In diesem Zustand spricht man typischerweise von einem "entblockten" Cuff. Typischerweise wird der Cuff insbesondere zum Einführen der Kanüle in die Trachea oder kurz vor der Entnahme einer Kanüle aus der Trachea entblockt.

Die erfindungsgemäße Beatmungsvorrichtung kommt bei bestimmungsgemäßem Gebrauch im Bereich des Stomaabschnitts an dem Stoma eines Patienten zum Anliegen. Ausgehend von dem Stomaabschnitt erstrecken sich in proximaler Richtung der Extrakorporalabschnitt und in distaler Richtung der Trachealabschnitt.

Die Begriffe "distal" und "proximal" werden im Sinne der vorliegenden Erfindung aus der Sicht eines die Beatmungsvorrichtung anwendenden Arztes verwendet, d. h. das proximale Ende ist das Ende der Beatmungsvorrichtung, das nach dem Einführen in die Trachea außerhalb des Patientenkörpers verbleibt, während das distale Ende in die Trachea des Patienten eingeführt wird.

Der Stomaabschnitt ist ein standardisierter Bereich, der je nach Größe und Halsdurchmesser des Patienten etwas länger (Erwachsene) oder etwas kürzer (Kinder) ausgestaltet ist. Dementsprechend variiert auch die Länge des Trachealabschnitts, der sich in distaler Richtung unmittelbar an den Stomaabschnitt anschließt.

Die Manschette bzw. der Cuff befindet sich bei der vorliegenden Erfindung im Trachealabschnitt vorzugsweise in einem Bereich vor dem distalen Ende des Kanülenrohrs. Je nach Größe und Halsdurchmesser des Patienten erstreckt sich der Bereich, in dem die Manschette bzw. der Cuff liegt, ausgehend vom distalen Ende des Kanülenrohrs in Richtung proximales Ende des Kanülenrohres über bis zu 50%, über bis zu 40%, über bis zu 30% oder nur über bis zu 20% der Gesamtlänge der zentralen Längsachse des Kanülenrohres. V.a. bei kürzeren Ausführungsformen für Kinder ist der Anteil des Bereichs an der Kanülenrohrlänge größer (z.B. 40 bis 50%) als bei längeren Ausführungsformen für Erwachsene (z.B. 20 bis 40%).

Der Begriff "Leitung" wird im Zusammenhang mit der vorliegenden Erfindung im Sinne einer fluidführenden Leitung verstanden, in die an dem wenigstens einen vorgesehenen Fluideinlass ein Fluid einströmen kann und aus der das Fluid an dem wenigstens einen vorgesehenen Fluidauslass wieder ausströmen kann, nachdem das Fluid durch die ansonsten fluiddichte Leitung über eine bestimmte Strecke hindurchgeströmt ist.

Die erfindungsgemäß vorgeschlagene Beatmungsvorrichtung ist mit dem Vorteil verbunden, dass sich der für das möglichst störungsfreie Hindurchleiten von Fluid erforderliche Querschnitt des Innenlumens der Fluid-Leitung vor allem dann ausbildet, wenn tatsächlich Fluid durch die Fluid-Leitung strömt. Insbesondere weist das variable Lumen in dem Abschnitt der Fluid-Leitung die erfindungsgemäß durch eine Folie gebildet wird in diesem Abschnitt n bei einem in der Manschette vorliegenden spezifischen Fülldruck mit einem Überdruck von 15-200 mbar bezogen auf den Umgebungsdruck (Atmosphärendruck) einen Querschnitt Qn₂ auf, der wenigstens um den Faktor 3 größer ist als der Querschnitt Qn₁, wenn in der Manschette bezogen auf den Umgebungsdruck (Atmosphärendruck) ein Unterdruck von -30 mbar vorliegt.

In den Phasen, in denen der Cuff geblock ist, stört es nicht, wenn die Fluid-Leitung einen etwas größeren Raum einnimmt, da die Luft zum Atmen bzw. zum Beatmen ausschließlich durch das Kanülenrohr fließt. Hier ist der Vorteil einer großen Fluid-Leitung größer als der Nachteil. In den Phasen, in denen der Cuff entblockt ist, ist es jedoch wichtig, dass die Fluid-Leitung möglichst wenig Raum einnimmt. Dies ist beispielsweise bei der Einführung der Kanüle der Fall. Aber auch in Phasen, in denen der Patient bei verschlossenem Kanülenrohr an der Kanüle vorbei durch die natürlichen Atemwege einatmen und/oder ausatmen soll. Hier würde ein zu großer Querschnitt der Fluid-Leitung dazu führen, dass der Atemwiderstand für den Patienten zu groß wird, um selbstständig am Kanülenrohr vorbei zu atmen. Die Konsequenz wäre zum Beispiel, dass der Patient nicht oder nur sehr schlecht sprechen könnte. Zusätzlich würden bestimmte Therapieformen zur Entwöhnung des Patienten von der Kanüle erschwert. Ein zu großer Außendurchmesser der Kanüle könnte somit dazu führen, dass der Patient nicht von der Kanüle entwöhnt werden kann und dauerhaft die Kanüle benötigt.

Dass sich der für das möglichst störungsfreie Hindurchleiten von Fluid erforderliche Querschnitt des Innenlumens der Fluid-Leitung vor allem dann ausbildet, wenn tatsächlich Fluid durch die Fluid-Leitung strömt, wird erfindungsgemäß dadurch erreicht, dass die Fluid-Leitung im Bereich des Stomaabschnitts und/oder des Trachealabschnitts zumindest abschnittsweise von einer auf der Außenseite des Kanülenrohrs angeordneten Folie ausgebildet wird, die ein Lumen definiert, dass unter den oben genannten Bedingungen in dem oben definierten Umfang variabel ist.

Der Begriff "Umgebungsdruck" bezeichnet im Sinne der vorliegenden Erfindung den auf die Folie wirkenden Druck. Bei einer nicht im Gebrauch befindlichen Beatmungsvorrichtung, die nicht in die Trachea eingeführt vorliegt und durch deren Fluid-Leitung kein Fluid, insbesondere keine Luft geführt wird, entspricht der Umgebungsdruck dem Atmosphärendruck und dieser wirkt gleichermaßen auf die nach außen und die nach innen gewandte Seite der Folie.

Wird der Cuff bzw. die Manschette bezogen auf den Umgebungsdruck (Atmosphärendruck) mit einem Überdruck von 15-200 mbar befüllt, wirkt auf die nach innen gewandte Seite der Folie ein entsprechend erhöhter Druck, der dazu führt, dass die Folie in einem von der Folie gebildeten Abschnitt n, einen Querschnitt Qn₂ aufweist. Wird die Cuff-Manschette allerdings entleert und wird dabei ein Unterdruck von -30 mbar angelegt, so verringert sich der Querschnitt Qn₁ in dem Abschnitt n erfindungsgemäß wenigstens um den Faktor 3.

Der Begriff "Querschnitt" bezeichnet im Zusammenhang mit der vorliegenden Erfindung die Fläche eines Querschnitts durch das Innenlumen der Cuff-Leitung senkrecht zur Hauptströmungsrichtung. Der Querschnitt Qn₁ des Lumens der erfindungsgemäßen Cuff-Leitung ist gleich die Fläche des Querschnitts durch das Innenlumen in einem von der Folie gebildeten Abschnitt n bei einem Unterdruck von -30 mbar. Der Querschnitt Qn₂ des Lumens der erfindungsgemäßen Cuff-Leitung ist gleich die Fläche des Querschnitts durch das Innenlumen in dem Abschnitt n bei einem in der Manschette und damit auch in der Leitung vorliegenden Überdruck von 15-200 mbar.

Gemessen wird der Querschnitt jeweils im freien Zustand, d.h. in einem Zustand, in dem sich die erfindungsgemäße Beatmungsvorrichtung, wie z.B. eine Tracheostomiekanüle oder eine Endotrachealkanüle, außerhalb des Körpers befindet. Bestimmt wird also der Querschnitt des Lumens "im freien Raum", d.h. ohne dass die Ausdehnung des Lumens durch andere Objekte in der Umgebung mechanisch beeinträchtigt wird.

Die Messung der Variabilität des Lumens eines von der Folie gebildeten Abschnitts der Fluid-Leitung unter den verschiedenen Druckbedingungen kann dadurch erfolgen, dass man den interessierenden Abschnitt der Fluid-Leitung bezogen auf den Umgebungsdruck (Atmosphärendruck) einmal mit einem Überdruck von 15-200 mbar mit einem Fluid befüllt und anschließend bezogen auf den Umgebungsdruck (Atmosphärendruck) einen Unterdruck von -30 mbar anlegt und den Querschnitt des Lumens unter den jeweiligen Druckbedingungen mit einem optischen Verfahren vermisst. Zur Bestimmung des Querschnitts des Lumens unter bestimmten Druckbedingungen könnte man alternativ die Leitung unter den interessierenden Bedingungen auch mit einem Harz ausgießen, das langsam erhärtet. Nach erfolgter Erhärtung können dann Querschnitte angefertigt werden und deren Dimensionen ermittelt werden. Eine weitere Methode wäre die Bestimmung des unter den verschiedenen Druckbedingungen im intessierenden Abschnitt der Leitung aufgenommenen Fluid-Volumens und die rechnerische Ermittlung der sich dadurch ergebenden Querschnitte.

Um beim Einführen der erfindungsgemäßen Beatmungsvorrichtung oder auch bei der Entnahme der Beatmungsvorrichtung insgesamt einen möglichst geringen Querschnitt der gesamten Beatmungsvorrichtung zu haben, wodurch das Bewegen des Kanülenrohrs durch Stoma und/oder Trachea erleichtert wird, weist der Querschnitt des von der Folie gebildeten Abschnitts n der Fluid-Leitung bei einem in der Manschette vorliegenden Unterdruck von -30 mbar bezogen auf den Umgebungsdruck (Atmosphärendruck) einen Querschnitt Qn₁ auf, der höchstens 0,3 mm im Quadrat beträgt.

Der Abschnitt n bezeichnet hier einen von der Folie gebildeten Abschnitt der Fluidleitung, der bei einem vorgegebenen Druck einen konstanten Querschnitt Qn₁ bzw. Qn₂ aufweist. Ist über die gesamte von der Folie gebildete Länge der Fluidleitung der Querschnitt Qn₁ bzw. Qn₂ konstant so erstreckt sich der Abschnitt n über diese gesamte Länge. Variiert der Querschnitt abschnittsweise bezieht sich n jeweils auf den Abschnitt in dem der Querschnitt konstant ist.

Bei bestimmten Ausführungsformen wird die Fluid-Leitung zumindest abschnittsweise von einer Lage der Folie gebildet, die auf der Außenseite der Rohrwand des Kanülenrohrs angeordnet ist. Vorzugsweise handelt es sich hierbei um eine einschichtige Lage der Folie, deren Längsseiten mit der Außenseite der Rohrwand des Kanülenrohrs fluiddicht verbunden sind, beispielsweise durch Verkleben, Einklemmen oder Verschweißen.

Bei manchen Ausführungsformen besteht die Folie aus einer 2- oder 3-schichtigen-Lage. Auf diese Weise kann beispielsweise eine innere Schicht der Lage in ihren Eigenschaften so gewählt werden, dass möglichst geringe Kohäsionsneigung für Wassertröpfchen vorliegt. Vorzugsweise kann eine äußere oder mittlere Schicht so gewählt sein, dass diese eine besonders hohe Reißfestigkeit und/oder besonders große mechanische Widerstandskraft aufweist.

Die Aufbringung der Folie in Form einer Lage auf die Außenseite der Rohrwand des Kanülenrohrs ist mit dem Vorteil verbunden, dass relativ wenig Folienmaterial verwendet werden muss. Zum anderen ergibt sich hierdurch eine typischerweise halbmondförmige Gestalt des Querschnitts des Lumens der Fluid-Leitung, wodurch ein Lumen erreicht werden kann, das in radialer Richtung relativ wenig aufträgt.

Bei speziellen Ausführungsformen ist die Lage der Folie auf der Außenseite der Rohrwand wenigstens abschnittsweise über den gesamten äußeren Querschnittsumfang des Kanülenrohrs vorgesehen. Die Lage der Folie bildet somit praktisch einen Schlauch durch den das Kanülenrohr verläuft, wobei das Fluid zum Befüllen des Cuffs dann zwischen diesem äußeren Folienschlauch und dem inneren Kanülenrohr strömen kann.

Bei manchen Ausführungsformen ist auf der Außenseite der Rohrwand des Kanülenrohrs in dem Bereich, der von der Lage der Folie überspannt wird, in Längsrichtung wenigstens eine rillenförmige Ausnehmung vorgesehen, die gemeinsam mit der Außenseite der Rohrwand des Kanülenrohrs und der Innenseite der den Bereich überspannenden Folie den Querschnitt definiert, durch den das Fluid zum Befüllen des Cuffs strömt. Das Lumen zwischen Folieninnenseite und Rohrau-ßenwand wird somit in Längsrichtung um den Querschnitt der wellenförmigen Ausnehmung erweitert.

Bei bestimmten Ausführungsformen ist die Fluid-Leitung zumindest abschnittsweise von einem aus der Folie bestehenden Schlauch gebildet, der auf der Außenseite der Rohrwand des Kanülenrohrs angeordnet ist. Der Schlauch ist bei diesen Ausführungsformen nicht so ausgeführt, dass er das gesamte Kanülenrohr umhüllt, sondern es ist ein Schlauch mit einem Querschnitt, der deutlich geringer ist als der Querschnitt des Kanülenrohrs, und dieser Schlauch ist an eine Seite des Kanülenrohrs in Längsrichtung an der Außenseite der Rohrwand angebracht, beispielsweise durch Aufkleben, Einklemmen und/oder Verschweißen.

Die Anbringung der Fluid-Leitung in Form eines aus der Folie bestehenden Schlauches hat den Vorteil, dass ein solcher Schlauch an sich eine große Zuverlässigkeit im Hinblick auf die Dichtigkeit mit sich bringt, während im Falle der Anbringung einer Lage der Folie auf der Außenseite der Kanülenwand die Verbindung fluiddicht erfolgen muss, um die erforderliche Dichtigkeit zu erreichen.

Vorzugsweise erstreckt sich der durch die Folie gebildete Abschnitt der Fluid-Leitung in der Richtung proximal-distal wenigstens über 50 %, wenigstens über 60 %, wenigstens über 70 %, wenigstens über 80 %, wenigstens über 90 % oder über 100 % des Abschnitts des Kanülenrohrs, der zwischen dem Extrakorporalabschnitt und der Cuff-Manschette liegt.

Bei manchen Ausführungsformen wird Qn₂ durch eine Dehnung der Folie erreicht, die sich bei einem Cuff-Überdruck von 15-200 mbar einstellt. Die Reduzierung des Querschnitts auf Qn₁ durch einen Unterdruck von -30 mbar stellt sich bei diesen Ausführungsformen zumindest teilweise dadurch ein, dass die Dehnung der Folie aufgrund ihrer Elastizität wieder zurückgeht.

Bei anderen Ausführungsformen wird Qn₂ dadurch erreicht, dass bei einem Überdruck von 15-200 mbar bezogen auf den Umgebungsdruck (Atmosphärendruck) die Folie entgegen der Schwerkraftwirkung bzw. entgegen der Eigenspannung der Folie aufgespannt wird, ohne dass hierbei eine Dehnung der Folie erfolgt. Die Reduzierung auf Qn₁ durch einen Unterdruck von -30 mbar stellt sich bei diesen Ausführungsformen dadurch ein, dass die Folie wenigstens teilweise zusammenfällt.

Bei speziellen Ausführungsformen stellt sich Qn₂ teilweise, beispielsweise zu 20-80 %, durch eine Dehnung der Folie und teilweise, beispielsweise zu 80-20 %, durch Aufspannen der Folie bei einem Überdruck von 15-200 mbar ein.

Bei bestimmten Ausführungsformen weisen die Folie bzw. der Folienschlauch eine vorgeformte Geometrie mit geringerem Querschnitt auf, wobei sich der vollständige Querschnitt erst dann ausbildet, wenn in der Fluid-Leitung ein Überdruck im Bereich von 15-200 mbar vorliegt. Bei entsprechend geringerem Druck nimmt die Folie bzw. der Schlauch wieder den Querschnitt der vorgeformten Geometrie ein. Die vorgeformte Geometrie kann beispielsweise eine oder mehrere in Längsrichtung verlaufende Einbuchtungen vorgeben. Dies kann beispielsweise durch unterschiedliche Wandstärken der Folie in den jeweiligen Bereichen bewirkt werden.

Bei manchen Ausführungsformen weist die Folie der Fluid-Leitung eine besonders hohe Reißfestigkeit auf und ist wenigstens bis zu einem Fluid-Druck von 250 mbar reißfest. Vorzugsweise weist die Folie der Fluid-Leitung eine Bruchdehnung nach DIN in ISO 527 im Bereich von 150-600 % auf.

Die Reißfestigkeit und auch die Variabilität des Lumens der Fluid-Leitung werden zum Teil durch das hierfür ausgewählte Kunststoffpolymermaterial bedingt. Ein weiterer Faktor ist jedenfalls zum Teil auch die Schichtdicke (Wandstärke) der Folie. Je dünner das Folienmaterial ist, desto anfälliger ist es typischerweise für Beschädigungen, wie z.B. der Risse. Dickeres Folienmaterial trägt wiederum stärker auf und ist insofern von Nachteil. Es ist daher ein Abwägen erforderlich, welches Kriterium wie gewichtet werden soll.

Je nach den speziellen Anforderungen und den speziellen Eigenschaften der Folie, die zur Ausbildung der Fluid-Leitung bei der vorliegenden Erfindung verwendet wird, kann die Wandstärke der Folie variieren. Besonders geeignet sind Wandstärken im Bereich von 5 bis 300 µm, vorzugsweise im Bereich von 10 bis 100 µm noch bevorzugter im Bereich von 10 bis 30 µm.

Typischerweise besteht die Folie der Fluid-Leitung aus einem Kunststoffpolymer. Als Kunststoffpolymere für die Bildung der Folie der Fluid-Leitung kommen verschiedene Materialien in Betracht, darunter Polyurethan, Weich-PVC, PE, PP, EVA, PEBAX und Silikon. Vorzugsweise handelt es sich um ein Kunststoffpolymer mit einem Shore A im Bereich von 5-90.

Typischerweise besteht auch die Manschette aus einer Kunststoffpolymerfolie. Bei bestimmten Ausführungsformen sind die Folie der Fluid-Leitung und die Folie der Manschette aus demselben Kunststoffpolymermaterial gebildet. Besonders bevorzugt sind Folie und Manschette bei diesen Ausführungsformen nahtlos ineinander übergehend bzw. einstückig ausgebildet.

Bei bestimmten Ausführungsformen ist die Fluid-Leitung im Bereich des Extrakorporalabschnitts aus einem flexiblen Kunststoffschlauch gebildet, der eine größere Härte und/oder Wandstärke aufweist, als die Folie, aus welcher die Fluid-Leitung zumindest abschnittsweise im Stomaabschnitt und/oder Trachealabschnitt besteht.

Für Zwecke der ursprünglichen Offenbarung wird darauf hingewiesen, dass sämtliche Merkmale, wie sie sich aus der vorliegenden Beschreibung, den anhängenden Zeichnungen und den Ansprüchen für einen Fachmann erschließen, auch wenn sie konkret nur im Zusammenhang mit bestimmten weiteren Merkmalen beschrieben wurden.

Auf die umfassende, explizite Darstellung sämtlicher denkbarer Merkmalskombinationen wird hier nur der Kürze und der Lesbarkeit der Beschreibung wegen verzichtet.

In den anhängenden Figuren und der dazugehörigen Figurenbeschreibung sind einzelne konkrete Ausführungsformen der Erfindung schematisch dargestellt. Diese konkreten Ausführungsformen sind lediglich ein Beispiel für mögliche Merkmalskombinationen. Die Erfindung ist jedoch keinesfalls auf diese konkreten Ausführungsformen beschränkt, sondern umfasst alle vom Schutzumfang der Patentansprüche abgedeckten Ausführungsformen, auch wenn sie nicht explizit dargestellt oder beschrieben sind.

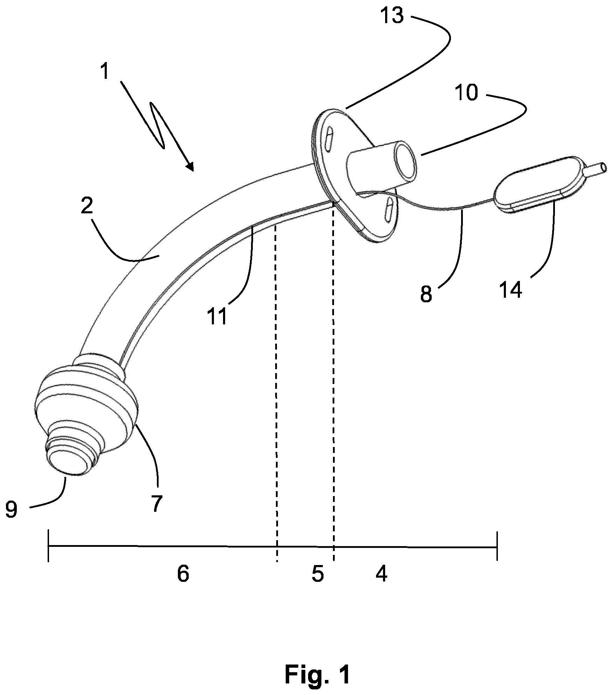

- Figur 1:: Figur 1 zeigt eine perspektivische Ansicht auf eine Ausführungsform einer erfindungsgemäßen Beatmungsvorrichtung, bei der die Fluid-Leitung zum Befüllen der Manschette im Stomaabschnitt und im Trachealabschnitt in Form einer Folienlage vorgesehen ist, während im Extrakorporalabschnitt die Fluid-Leitung in Form eines Schlauches fortgesetzt ist.

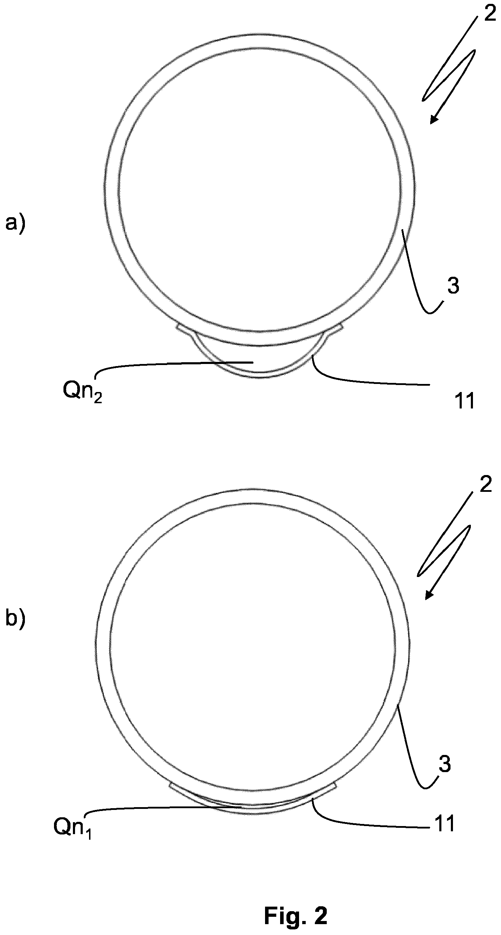

- Figur 2:: Die Abbildungen a) und b) von Figur 2 stellen einen schematischen Querschnitt durch das Kanülenrohr im Trachealabschnitt einer Ausführungsform einer erfindungsgemäßen Beatmungsvorrichtung dar, bei der die Fluid-Leitung zum Befüllen der Manschette in Form einer auf der Außenseite des Kanülenrohrs angeordneten Folienlage realisiert ist.

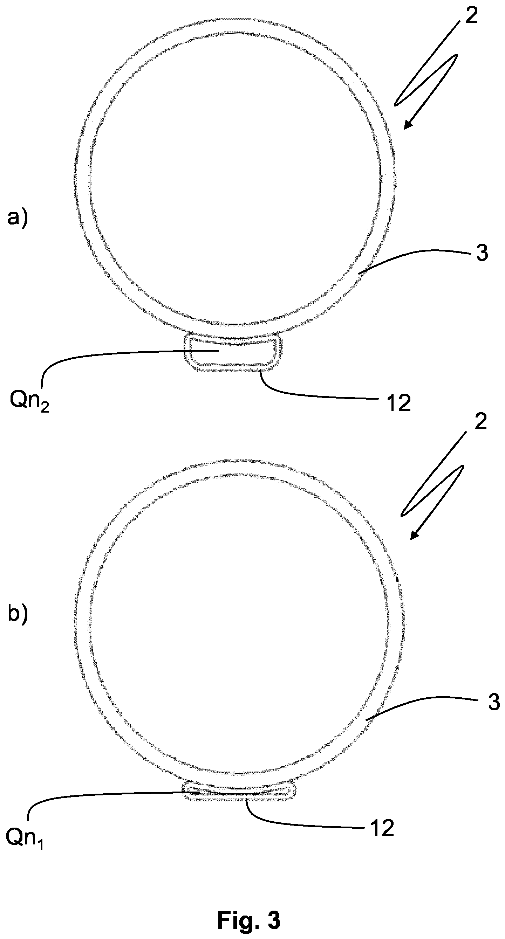

- Figur 3:: Die Abbildungen a) und b) von Figur 3 stellen einen schematischen Querschnitt durch das Kanülenrohr im Trachealabschnitt einer Ausführungsform einer erfindungsgemäßen Beatmungsvorrichtung dar, bei der die Fluid-Leitung zum Befüllen der Manschette in Form eines auf der Außenseite des Kanülenrohrs angeordneten Folienschlauchs realisiert ist.

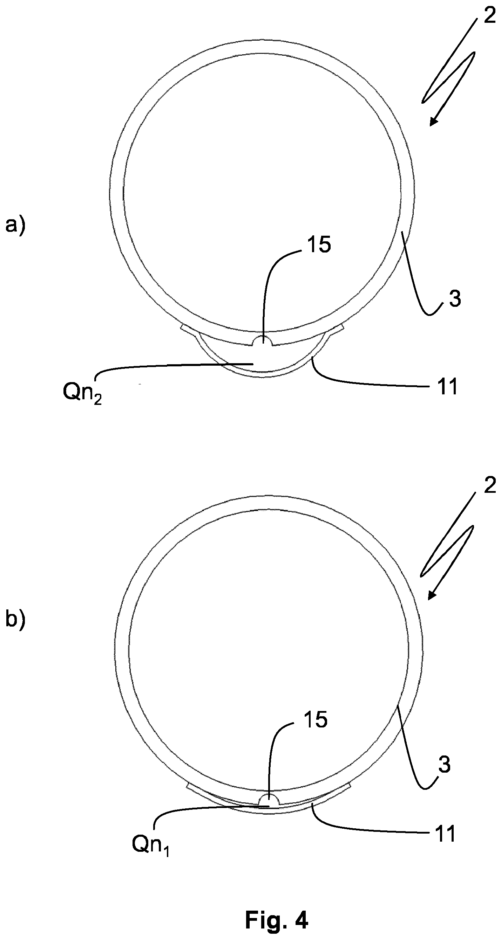

- Figur 4:: Die Abbildungen a) und b) von Figur 4 stellen einen schematischen Querschnitt durch das Kanülenrohr im Trachealabschnitt einer Ausführungsform einer erfindungsgemäßen Beatmungsvorrichtung dar, bei der die Fluid-Leitung zum Befüllen der Manschette in Form einer Folienlage realisiert ist, wobei der Querschnitt des Lumens, das sich zwischen Folienlage und Außenseite des Kanülenrohrs ergibt, durch eine in der Wand des Kanülenrohrs vorgesehene rillenförmige Ausnehmung erweitert ist.

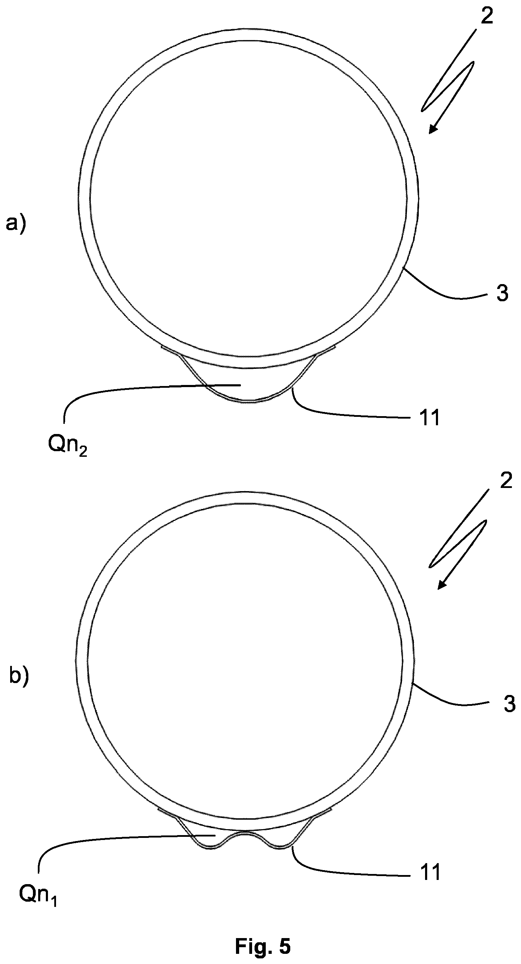

- Figur 5:: Die Abbildungen a) und b) von Figur 5 stellen einen schematischen Querschnitt durch das Kanülenrohr im Trachealabschnitt einer Ausführungsform einer erfindungsgemäßen Beatmungsvorrichtung dar, bei der die Fluid-Leitung zum Befüllen der Manschette in Form einer auf der Außenseite des Kanülenrohrs angeordneten vorgespannten Folienlage realisiert ist.

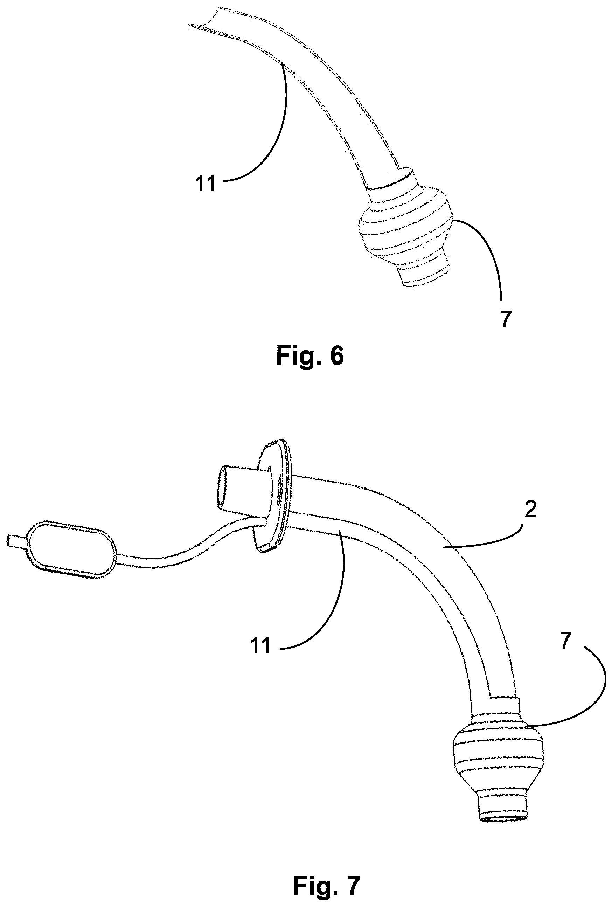

- Figur 6:: In Figur 6 ist eine zur Ausbildung einer Fluid-Leitung zum Befüllen der Manschette bestimmte Folienlage dargestellt, die nahtlos in das Material der Manschette übergeht.
- Figur 7:: Die in Figur 7 dargestellte Beatmungsvorrichtung zeigt die in Figur 6 gezeigte einstückig ausgeführte Kombination von Folienlage und Manschette nach der Montage auf dem Kanülenrohr einer bestimmten Ausführungsform einer erfindungsgemäßen Beatmungsvorrichtung.

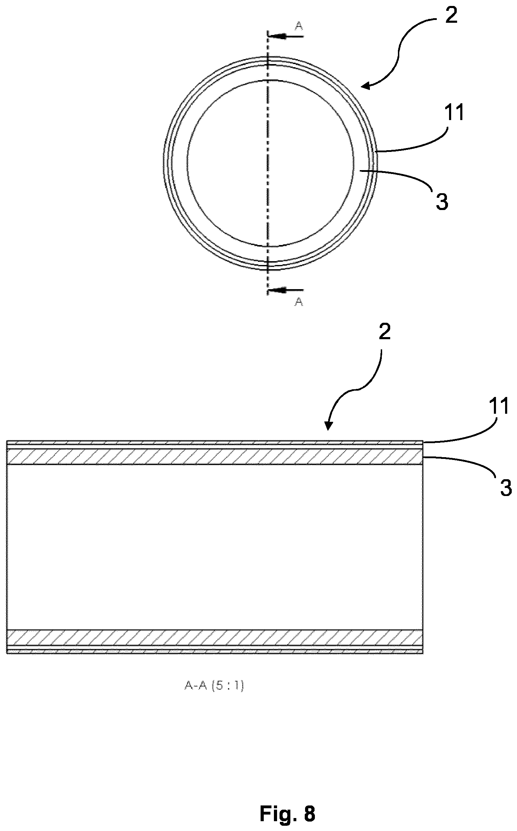

- Figur 8:: Bei der in Figur 8 dargestellten Ausführungsform der erfindungsgemäßen Beatmungsvorrichtung ist die Fluid-Leitung zum Befüllen der Manschette dadurch gebildet, dass sich eine Lage der Folie auf der Außenseite der Rohrwand in dem hier dargestellten Abschnitt über den gesamten äußeren Querschnittsumfang des Kanülenrohrs erstreckt.

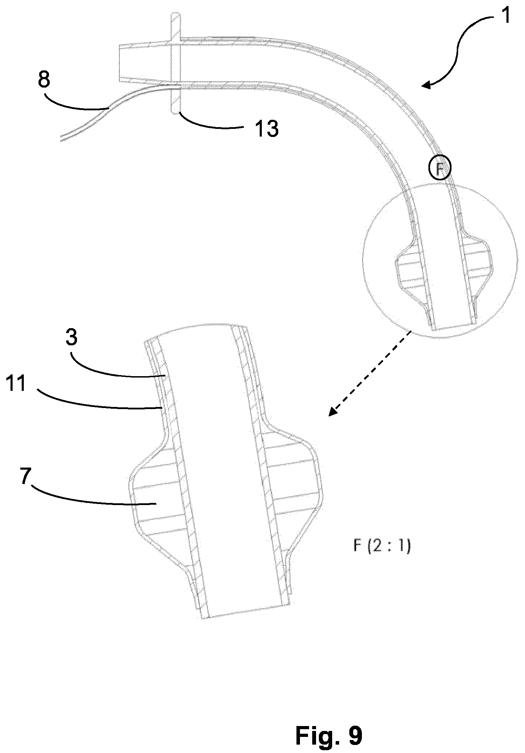

- Figur 9:: In Figur 9 ist eine Sonderform der in Figur 8 dargestellten Ausführungsform gezeigt, bei der die sich über den gesamten äußeren Querschnittsumfang des Kanülenrohrs erstreckende Folienlage nahtlos in die Manschette übergeht.

In Figur 1 ist als Beispiel für eine erfindungsgemäße Beatmungsvorrichtung eine Tracheostomiekanüle 1 dargestellt, deren Kanülenrohr einen Extrakorporalabschnitt 4, einen Stomaabschnitt 5 und einen Trachealabschnitt 6 aufweist. Der Extrakorporalabschnitt 4 ist der Bereich, der sich ab dem Kanülenschild 13 in proximale Richtung bis zum proximalen Ende der Kanüle erstreckt. In distaler Richtung schließt sich an das Kanülenschild 13 zunächst der Stomaabschnitt 5 an, direkt gefolgt vom Trachealabschnitt 6. Der Stomaabschnitt 5 ist ein standardisierter Bereich, der je nach Größe und Halsdurchmesser des Patienten etwas länger (Erwachsene) oder etwas kürzer (Kinder) ausgestaltet ist. Dementsprechend variiert auch die Länge des Trachealabschnitts 6, der sich in distaler Richtung unmittelbar an den Stomaabschnitt 5 anschließt.

Das Kanülenrohr 2 weist eine proximale Öffnung 10 und eine distale Öffnung 9 auf, und am distalen Ende des Kanülenrohrs 2 ist eine aufpumpbare Manschette 7 angeordnet, die hier im aufgepumpten Zustand dargestellt ist. Die Befüllung der Manschette 7 erfolgt über eine Fluid-Leitung, die bei der hier dargestellten Ausführungsform extrakorporal in Form eines Fluid-Schlauchs 8 und ab dem Kanülenschild 13 in Form einer auf dem Kanülenrohr 2 aufgebrachten Folienlage 11 vorgesehen ist. Die Folienlage ist bei der hier dargestellten Ausführungsform fluiddicht auf das Kanülenrohr aufgeklebt. Am distalen Ende mündet das von der zwischen der Folienlage 11 und der Außenseite des Kanülenrohrs 2 gebildete Lumen in das Lumen der Manschette 7, sodass am proximalen Ende in die Fluid-Leitung eingeführtes Fluid in die Manschette 7 gelangt, wodurch diese aufgepumpt werden kann. Am proximalen Ende des extrakorporalen Fluid-Schlauchs 8 ist ein Kontrollballon 14 angeordnet, über den haptisch und/oder visuell kontrolliert werden kann, ob die Manschette 7 aufgepumpt ist oder nicht. Am Ende des Kontrollballons ist dann ein Füllventil angebracht.

Figur 2 zeigt einen schematischen Querschnitt durch das Kanülenrohr 2 eine Ausführungsform einer erfindungsgemäßen Beatmungsvorrichtung im Trachealabschnitt. Die Rohrwand 3 des Kanülenrohrs 2 definiert in diesem Bereich einen kreisrunden Kanülenrohrinnenraum, durch den die Atemluft strömen kann. Auf der Außenseite des Kanülenrohrs 2 ist auf der Rohrwand 3 eine flexible Kunststofffolienlage 11 angeordnet, die bei erfindungsgemäß erhöhtem Innendruck in dem hier dargestellten Abschnitt ein Lumen mit einem Querschnitt Qn₂ gut definiert (Abbildung a)). In der Abbildung b) ist der Zustand dargestellt, in dem der erfindungsgemäße Unterdruck vorliegt, wodurch sich der Querschnitt Qn₁ entsprechend reduziert. Bereits mit bloßem Auge ist hierzu erkennen, dass der Querschnitt Qn₂ bei der hier dargestellten Ausführungsform wenigstens um den Faktor 3 größer ist als der Querschnitt Qn₁. Je nach Ausführungsform könnten sich beim Anlegen von Unterdruck auch Falten ausbilden

In Figur 3 ist ein schematischer Querschnitt durch das Kanülenrohr 2 eine Ausführungsform einer erfindungsgemäßen Beatmungsvorrichtung im Trachealabschnitt dargestellt, wobei hier auf der Außenseite des Kanülenrohrs 2 auf der Rohrwand 3 ein flexibler Folienschlauch 12 angeordnet ist, der bei erfindungsgemäß erhöhtem Innendruck ein Lumen mit einem Querschnitt Qn₂ definiert (Abbildung a)), während bei erfindungsgemäß angelegtem Unterdruck der Querschnitt Qn₁ des Folienschlauchs 12 wenigstens um den Faktor 3 geringer ist (Abbildung b)). Die Folienschlauch 12 ist bei der hier dargestellten Ausführungsform fluiddicht auf das Kanülenrohr 2 aufgeklebt.

In Figur 4 ist eine besondere Ausführungsform der vorliegenden Erfindung dargestellt, bei der das durch die Folienlage 11 in Verbindung mit der Außenseite der Rohrwand 3 des Kanülenrohrs 2 gebildete Lumen im Vergleich zu der in Figur 2 dargestellten Ausführungsform dadurch vergrößert ist, dass in der Rohrwand 3 eine rinnenförmige Ausnehmung 15 vorgesehen ist, die in Längsrichtung des Kanülenrohrs 2 auf der Außenseite der Rohrwand 3 verläuft. Die Folienlage 11 ist bei der hier dargestellten Ausführungsform fluiddicht auf das Kanülenrohr 2 aufgeklebt.

In Figur 5 ist eine auch besondere Ausführungsform einer erfindungsgemäßen Beatmungsvorrichtung dargestellt, bei der die Folie der Folienlage 11, die hier im Zusammenspiel mit der Außenseite der Rohrwand 3 des Kanülenrohrs 2 die Fluid-Leitung bildet eine vorgeformte Geometrie aufweist, die sich ausbildet, wenn kein Überdruck in der Fluid-Leitung oder sogar Unterdruck vorliegt. Bei der hier dargestellten Ausführungsform ist die vorgeformte Geometrie so ausgestaltet, dass in Längsrichtung der Fluid-Leitung mittig eine durchgehende Einbuchtung gebildet wird (Abbildung b)). Bei Anlegen eines Überdrucks im erfindungsgemäßen Bereich wird diese Einbuchtung nach außen ausgestülpt und hierdurch erhöht sich der Querschnitt des Lumens der Fluid-Leitung entsprechend (Abbildung a)). Die Folienlage 11 ist bei der hier dargestellten Ausführungsform fluiddicht auf das Kanülenrohr 2 aufgeschweißt.

Die Figuren 6 und 7 veranschaulichen eine spezielle Ausführungsform der erfindungsgemäßen Beatmungsvorrichtung, bei der die für die Ausbildung der Fluid-Leitung vorgesehene Folienlage 11 nahtlos in die Manschette 7 übergeht und somit mit dieser einstückig ausgebildet ist. In Figur 6 ist die Folienlage 11 zusammen mit der Manschette 7 separat, d. h. im nicht montierten Zustand dargestellt. In Figur 7 ist dann der Zustand dargestellt, in dem die Folienlage 11 auf dem Kanülenrohr aufgebracht ist und somit auch die Manschette 7. Die Folienlage und die Manschette sind bei der hier dargestellten Ausführungsform fluiddicht auf das Kanülenrohr aufgeschweißt.

Figur 8 ist eine schematische Darstellung einer besonderen Ausführungsform der vorliegenden Erfindung, bei der die Fluid-Leitung durch eine Folienlage 11 gebildet wird, die auf der Außenseite der Rohrwand 3 des Kanülenrohrs 2 über den gesamten äußeren Querschnittsumfang des Kanülenrohrs verläuft, sodass sich praktisch ein Schlauch ergibt, durch den das Kanülenrohr 2 verläuft, wobei das Fluid zum Befüllen der Manschette dann zwischen diesem äußeren Folienschlauch und dem darin befindlichen Kanülenrohr strömen kann.

In Figur 9 ist eine Variante der in Figur 8 dargestellten Ausführungsform der Erfindung gezeigt. Bei dieser Variante geht der über den gesamten Querschnittsumfang verlaufende Folienschlauch nahtlos in das Material der Manschette 7 über, sodass der von der Folienlage 11 gebildete Schlauch mit der Manschette 7 einstückig ausgebildet ist. In den zwischen der Folienlage 11 und der Außenseite der Rohrwand 3 des Kanülenrohrs 2 gebildeten Bereich der Fluid-Leitung mündet der extrakorporal angeordnete Folienschlauch 8 im Bereich des Kanülenschildes 13.

### Bezugszeichen

- 1: Beatmungsvorrichtung
- 2: Kanülenrohr
- 3: Rohrwand
- 4: Extrakorporalabschnitt
- 5: Stomaabschnitt
- 6: Trachealabschnitt
- 7: Manschette
- 8: extrakorporale Fluid-Leitung
- 9: distale Öffnung
- 10: proximale Öffnung
- 11: Folienlage (intrakorporale Fluid-Leitung)
- 12: Folienschlauch (intrakorporale Fluid-Leitung)
- 13: Kanülenschild
- 14: Kontrollballon mit Füllventil
- 15: rillenförmige Ausnehmung
- Qn: Querschnitt im Abschnitt n

## Patentansprüche

1. Beatmungsvorrichtung (1), insbesondere Tracheostomiekanüle, mit
▪ einem Kanülenrohr (2), dessen Rohrwand (3) einen Extrakorporalabschnitt (4), einen Stomaabschnitt (5) und einen Trachealabschnitt (6) aufweist,
▪ einer aufpumpbaren Manschette (7), die sich im Trachealabschnitt (6) an der Außenseite der Rohrwand (3) ringförmig um das Kanülenrohr (2) erstreckt, wobei die Manschette (7) ein variables Lumen zur Aufnahme eines Fluids aufweist, und
▪ einer Fluid-Leitung, die eine Öffnung in das Lumen der Manschette (7) aufweist,
wobei die Fluid-Leitung im Bereich des Stomaabschnitts (5) und/oder des Trachealabschnitts (6) zumindest abschnittsweise von einer auf der Außenseite des Kanülenrohres (2) angeordneten Folie (11, 12) gebildet wird, die ein variables Lumen für die Durchführung von Fluid ausbildet, **dadurch gekennzeichnet, dass** das variable Lumen eines von der Folie (11, 12) gebildeten Abschnitts n der Fluid-Leitung bei einem spezifischen Fülldruck zum Blocken der Manschette im Bereich von 15 bis 200 mbar Überdruck bezogen auf den Umgebungsdruck (Atmosphärendruck) einen Querschnitt Qn₂ aufweist, der wenigstens um den Faktor 3 größer ist als der Querschnitt Qn₁, wenn in der Manschette (7) bezogen auf den Umgebungsdruck (Atmosphärendruck) ein Unterdruck von -30 mbar vorliegt, wobei der Begriff Querschnitt die Fläche eines Querschnitts durch das Innenlumen der Cuff-Leitung senkrecht zur Hauptströmungsrichtung bezeichnet.

2. Vorrichtung nach Anspruch 1, **dadurch gekennzeichnet, dass** der Querschnitt des von der Folie (11, 12) gebildeten Abschnitts n der Fluid-Leitung bei einem in der Manschette vorliegenden Unterdruck von -30 mbar bezogen auf den Umgebungsdruck (Atmosphärendruck) einen Querschnitt Qn₁ aufweist, der höchstens 0,3 mm² beträgt.

3. Vorrichtung nach einem der Ansprüche 1 und 2, **dadurch gekennzeichnet, dass** die Fluid-Leitung zumindest abschnittsweise von einer Lage (11) der Folie gebildet wird, die auf der Außenseite der Rohrwand des Kanülenrohres angeordnet ist.

4. Vorrichtung nach Anspruch 3, **dadurch gekennzeichnet, dass** sich die Lage (11) der Folie auf der Außenseite der Rohrwand wenigstens abschnittsweise über den gesamten äußeren Querschnittsumfang des Kanülenrohres erstreckt.

5. Vorrichtung nach einem der Ansprüche 3 und 4, **dadurch gekennzeichnet, dass** auf der Außenseite der Rohrwand des Kanülenrohres in dem Bereich, der von der Lage (11) der Folie überspannt wird, in Längsrichtung wenigstens eine rillenförmige Ausnehmung (15) vorgesehen ist.

6. Vorrichtung nach einem der Ansprüche 1 und 2, **dadurch gekennzeichnet, dass** die Fluid-Leitung zumindest abschnittsweise von einem aus der Folie bestehenden Schlauch (12) gebildet wird, der auf der Außenseite der Rohrwand des Kanülenrohres angeordnet ist.

7. Vorrichtung nach einem der Ansprüche 1 bis 6, **dadurch gekennzeichnet, dass** die Folie der Fluid-Leitung eine Wandstärke im Bereich von 5 bis 300 µm aufweist, vorzugsweise im Bereich von 10 bis 30 µm.

8. Vorrichtung nach einem der Ansprüche 1 bis 7, **dadurch gekennzeichnet, dass** die Folie der Fluid-Leitung wenigstens bis zu einem Fluid-Druck von 250 mbar reißfest ist.

9. Vorrichtung nach einem der Ansprüche 1 bis 8, **dadurch gekennzeichnet, dass** die Folie der Fluid-Leitung eine Bruchdehnung (nach DIN EN ISO 527) im Bereich von 150-600% aufweist.

10. Vorrichtung nach einem der Ansprüche 1 bis 9, **dadurch gekennzeichnet, dass** die Folie der Fluid-Leitung aus einem Kunststoffpolymer mit einem Shore A im Bereich von 5 bis 90 besteht.

11. Vorrichtung nach einem der Ansprüche 1 bis 10, **dadurch gekennzeichnet, dass** die Folie der Fluid-Leitung aus einem Kunststoffpolymer besteht, das ausgewählt ist unter Polyurethan, Weich-PVC, PE, PP, EVA, PEBAX und Silikon.

12. Vorrichtung nach einem der Ansprüche 1 bis 11, **dadurch gekennzeichnet, dass** die Folie der Fluid-Leitung und die Manschette aus demselben Kunststoffpolymer einstückig ausgebildet sind.

13. Vorrichtung nach einem der Ansprüche 1 bis 12, **dadurch gekennzeichnet, dass** die Fluid-Leitung im Bereich des Extrakorporalabschnitts aus einem flexiblen Kunststoffschlauch besteht, der eine größere Härte und/oder Wandstärke aufweist, als die Folie, aus der die Fluid-Leitung zumindest abschnittsweise im Stomaabschnitt und/oder Trachealabschnitt besteht.

## Claims

1. Ventilation device (1), in particular a tracheostomy cannula, having
■ a cannula tube (2), the tube wall (3) of which has an extracorporeal portion (4), a stoma portion (5) and a tracheal portion (6),
■ an inflatable cuff (7) which extends annularly around the cannula tube (2) in the tracheal portion (6), on the outer side of the tube wall (3), the cuff (7) having a variable lumen for receiving a fluid, and
■ a fluid line which has an opening into the lumen of the cuff (7),
wherein the fluid line, in the region of the stoma portion (5) and/or of the tracheal portion (6), is formed at least in part by a film (11, 12) which is arranged on the outer side of the cannula tube (2) and which forms a variable lumen for the passage of fluid, **characterized in that** the variable lumen of a portion n of the fluid line formed by the film (11, 12) has, at a specific filling pressure for blocking the cuff in the range of 15 to 200 mbar overpressure relative to the ambient pressure (atmospheric pressure), a cross section Qn₂ which is at least a factor of 3 larger than the cross section Qn₁ when an underpressure of -30 mbar relative to the ambient pressure (atmospheric pressure) is present in the cuff (7), the term "cross section" referring to the area of a cross section through the inner lumen of the cuff line perpendicular to the main direction of flow.

2. Device according to Claim 1, **characterized in that** the cross section of the portion n of the fluid line formed by the film (11, 12) has a cross section Qn₁ of not more than 0.3 mm² when there is an underpressure in the cuff of -30 mbar relative to the ambient pressure (atmospheric pressure).

3. Device according to either of Claims 1 and 2, **characterized in that** the fluid line is formed at least in part by a layer (11) of the film that is arranged on the outer side of the tube wall of the cannula tube.

4. Device according to Claim 3, **characterized in that** the layer (11) of the film on the outer side of the tube wall extends, at least in part, over the entire outer cross-sectional circumference of the cannula tube.

5. Device according to either of Claims 3 and 4, **characterized in that** at least one groove-shaped recess (15) is provided in the longitudinal direction on the outer side of the tube wall of the cannula tube in the region spanned by the layer (11) of the film.

6. Device according to either of Claims 1 and 2, **characterized in that** the fluid line is formed, at least in part, by a hose (12) which consists of the film and which is arranged on the outer side of the tube wall of the cannula tube.

7. Device according to any one of Claims 1 to 6, **characterized in that** the film of the fluid line has a wall thickness in the range of 5 to 300 µm, preferably in the range of 10 to 30 µm.

8. Device according to any one of Claims 1 to 7, **characterized in that** the film of the fluid line is tear-resistant at least up to a fluid pressure of 250 mbar.

9. Device according to any one of Claims 1 to 8, **characterized in that** the film of the fluid line has an elongation at break (according to DIN EN ISO 527) in the range of 150-600%.

10. Device according to any one of Claims 1 to 9, **characterized in that** the film of the fluid line consists of a plastic polymer having a Shore A in the range of 5 to 90.

11. Device according to any one of Claims 1 to 10, **characterized in that** the film of the fluid line consists of a plastic polymer selected from polyurethane, soft PVC, PE, PP, EVA, PEBAX and silicone.

12. Device according to any one of Claims 1 to 11, **characterized in that** the film of the fluid line and the cuff are made in one piece from the same plastic polymer.

13. Device according to any one of Claims 1 to 12, **characterized in that** the fluid line, in the region of the extracorporeal portion, consists of a flexible plastic hose which has a greater hardness and/or wall thickness than the film from which the fluid line is made, at least in part, in the stoma portion and/or tracheal portion.

## Revendications

1. Dispositif de ventilation (1), notamment canule de trachéotomie, avec
■ un tube de canule (2), dont la paroi de tube (3) présente une section extracorporelle (4), une section stomatique (5) et une section trachéale (6),
■ un manchon gonflable (7) s'étendant de manière annulaire autour du tube de canule (2) dans la section trachéale (6) sur le côté extérieur de la paroi de tube (3), le manchon (7) présentant une lumière variable pour recevoir un fluide, et
■ une conduite de fluide présentant une ouverture dans la lumière du manchon (7),
la conduite de fluide étant formée dans la zone de la section stomatique (5) et/ou de la section trachéale (6) au moins par sections par une feuille (11, 12) agencée sur le côté extérieur du tube de canule (2), qui réalise une lumière variable pour le passage de fluide, **caractérisé en ce que** la lumière variable d'une section n de la conduite de fluide formée par la feuille (11, 12) présente, à une pression de remplissage spécifique pour bloquer le manchon dans la plage de 15 à 200 mbar de surpression par rapport à la pression ambiante (pression atmosphérique), une section transversale Qn₂ qui est au moins 3 fois plus grande que la section transversale Qn₁, lorsqu'une dépression de -30 mbar par rapport à la pression ambiante (pression atmosphérique) est présente dans le manchon (7), le terme section transversale désignant la surface d'une section transversale à travers la lumière intérieure de la conduite de ballonnet perpendiculairement à la direction d'écoulement principale.

2. Dispositif selon la revendication 1, **caractérisé en ce que** la section transversale de la section n de la conduite de fluide formée par la feuille (11, 12) présente une section transversale Qn₁ qui est d'au plus 0,3 mm² à une dépression de -30 mbar présente dans le manchon par rapport à la pression ambiante (pression atmosphérique).

3. Dispositif selon l'une quelconque des revendications 1 et 2, **caractérisé en ce que** la conduite de fluide est formée au moins par sections par une couche (11) de la feuille agencée sur le côté extérieur de la paroi de tube du tube de canule.

4. Dispositif selon la revendication 3, **caractérisé en ce que** la couche (11) de la feuille sur le côté extérieur de la paroi de tube s'étend au moins par sections sur toute la circonférence de section transversale extérieure du tube de canule.

5. Dispositif selon l'une quelconque des revendications 3 et 4, **caractérisé en ce qu'**au moins un évidement (15) en forme de rainure est prévu dans la direction longitudinale sur le côté extérieur de la paroi de tube du tube de canule dans la zone qui est recouverte par la couche (11) de la feuille.

6. Dispositif selon l'une quelconque des revendications 1 et 2, **caractérisé en ce que** la conduite de fluide est formée au moins par sections par un tuyau (12) constitué de la feuille, qui est agencé sur le côté extérieur de la paroi de tube du tube de canule.

7. Dispositif selon l'une quelconque des revendications 1 à 6, **caractérisé en ce que** la feuille de la conduite de fluide présente une épaisseur de paroi dans la plage de 5 à 300 µm, de préférence dans la plage de 10 à 30 µm.

8. Dispositif selon l'une quelconque des revendications 1 à 7, **caractérisé en ce que** la feuille de la conduite de fluide est résistante à la déchirure au moins jusqu'à une pression de fluide de 250 mbar.

9. Dispositif selon l'une quelconque des revendications 1 à 8, **caractérisé en ce que** la feuille de la conduite de fluide présente un allongement à la rupture (selon la norme DIN EN ISO 527) dans la plage de 150 à 600 %.

10. Dispositif selon l'une quelconque des revendications 1 à 9, **caractérisé en ce que** la feuille de la conduite de fluide est constituée d'un polymère plastique ayant une dureté Shore A dans la plage de 5 à 90.

11. Dispositif selon l'une quelconque des revendications 1 à 10, **caractérisé en ce que** la feuille de la conduite de fluide est constituée d'un polymère plastique choisi parmi le polyuréthane, le PVC souple, le PE, le PP, l'EVA, le PEBAX et la silicone.

12. Dispositif selon l'une quelconque des revendications 1 à 11, **caractérisé en ce que** la feuille de la conduite de fluide et le manchon sont réalisés d'un seul tenant dans le même polymère plastique.

13. Dispositif selon l'une quelconque des revendications 1 à 12, **caractérisé en ce que** la conduite de fluide est constituée, dans la zone de la section extracorporelle, d'un tuyau en plastique flexible qui présente une dureté et/ou une épaisseur de paroi supérieure à celle de la feuille dont est constituée la conduite de fluide, au moins par sections, dans la section stomatique et/ou la section trachéale.
